Europäisches Patentamt

European Patent Office

Office européen des brevets

(11) Publication number: **0 255 902**
**A1**

# EUROPEAN PATENT APPLICATION

(21) Application number: 87110743.9

(51) Int. Cl.⁴: **A61K 47/00**

(22) Date of filing: 24.07.87

(30) Priority: 07.08.86 IT 2143986

(43) Date of publication of application:
**17.02.88 Bulletin 88/07**

(84) Designated Contracting States:
**AT BE CH DE ES FR GB GR LI LU NL SE**

(71) Applicant: **TOFCO SA**
**Via Maraini 7**
**Lugano(CH)**

(72) Inventor: **Ferro, Antonio**
**Via Maraini 7**
**CH-Lugano(CH)**
Inventor: **Gazzani, Giovanni**
**Via Volta 28**
**I-Appiano Gentile (Como)(IT)**

(74) Representative: **Dragotti, Gianfranco**
**SAIC BREVETTI s.a.s. Viale Bianca Maria, 15**
**I-20122 Milano(IT)**

(54) Composition for topical use.

(57) A composition for topical use, particularly having spermicide, virulicide, disinfectant activity, comprises a partially pre-hydrated vehicle capable of readily absorbing biological fluids, together with one or more substances having the activities of the above specified type.

The composition according to the invention are particularly effective as chemical prophylactics against the transmission through biological fluids of viruses responsible of Herpes genitalis and of the virus responsible of the disease called AIDS.

EP 0 255 902 A1

## "Composition for topical use"

The present invention relates to compositions for local use, both for topical use and for use into contact with the vaginal and rectal mucosae, having virulicide, spermicide, disinfectant and/or bactericide activity. More particularly the compositions according to the present invention find use as prophylactics suitable to prevent the transmission through biological fluids (mucous, sperms, serum and blood, urine) of virus responsible of nervous and dermal phenomena involving the male and female genital organs, known under the name of Herpes genitalis (JAMA 248, 9, pagg. 1041-1049-1982) as well as of the HTLV III virus responsible of the heavy immunodeficiencies known under the abbreviations AIDS (The New England Journal of Medicine, 313, 24 pagg. 1498-1510, 1985).

It is has been already found that the use of particular hydrophilic and water swellable polymeric substances together with suitable vehicles permits the provision both of vaginal tables having local spermicide action and of tablets which upon contacting the water form in short times extemporaneous gels and creams for cutaneous use having the advantage of the stability and of a practical and convenient use.

For the vaginal tablets (Italian Patent Application No. 22910 A/80) the use of particular modified dextrane is foreseen.

For the tablets giving place to extemporaneous gels and creams with water (Italian Patent Application No. 19284 A/86) the use is foreseen of particular water swellable polymers which are suitably associated with permeating substances avoiding the forming of layers.

In any case recourse is always made to only one form in dry state, namely the tablets having the most suitable forms.

It is to be pointed out that the rate of absorption of biological liquids may be determining in the case in which the inactivation time is in essential condition for the efficacy and safety of use, particularly of spermicides and disinfectants.

In these cases it is important to remove as much quickly as possible the spermatozoa, germs and virus which are potentially infectious and which are into contact with the mucosae and may escape the action of the inactivating substances.

Although for tablets forming extemporaneous creams and gel the absorption rate of the forming water may be reasonably delayed, in the case of spermicides and vaginal disinfectants the immediate absorption and consequently inactivation is an essential condition for a higher reliability.

For this reason the efforts aiming at obtaining a substantial increase of the absorption rate of the biological liquids have been exclusively devoted to the preparations for topical use on the mucosae wherein any biological extraneous presence is to be removed.

Surprisingly it has now demonstrated that the absorption rate of water and/or biological liquids increases in a remarkable manner, if instead of using an exclusively dry state formulation, an adequately prehydrated mixture is used.

The recourse to the controledl prehydration in order to increase the absorption rate of biological liquids, permits to attain times which are three to five times shorter than those for the dry state form.

By dosing the water according to well determined criteria it is possible to provide several forms such as tablets, granulates, microtablets, formulations of gels and creams having a reduced water content which are in form of powders or, in the case that the water content is close to the upper limits, in the form of viscous paste.

Of course, the final amount of absorbed water shall be less but the absorption shall take place in much less time.

If for instance a one gram tablet does absorb 10 ml of biological liquids in 60 seconds, the same tablet which has been already previously added with 2 ml water shall absorb 8 ml of biological liquids in 20 seconds. This finding permitted to provide spermicide and germicide products of greater safety coefficient. As a matter of fact these formulations, upon contacting the mucosae, immediately draw the present liquids and, if suitably added with spermicide and germicide substances, immediately inactivate any living form present therein.

They essentially operate as traps for every biological presence to be eliminated.

The active drawing permits any living form to be caught and blocked even when it is not directly into contact with the spermicide and micro bicide active substances.

In view of the remarkable capability of captating liquids of some derivatives such as modified dextrane (describe in the Italian Application No. 22910 A/80) and of some natural semisynthetic polymers (referred to in the Italian Patent Application No. 19284 A/86) the water added to the formulation may vary within rather wide limits as a function of the form to be realized without compromising the use reliability.

As a matter of fact when it is desired:

-to formulate granulates, tablets and microtablets, the constitution water may range from 10 to 100% on dry basis.

-to formulate concentrated creams ans gels with the aforesaid physical features, the water content may vary from 100 to 1000% on dry basis Even if all these pharmaceutical forms can be suitably used as vehicles adapted for spermicide, bactericide, antimicotic, anti-yeast, anti protozoa, a particular interest pertains to the possibility of achieving a valid also anti-viral prophylaxis when such a type of infection is transmitted through heterosexual and homosexual contacts.

More particularly there are two virus strains for which it is possible to prevent the infection:

-the HTLV III virus which is responsible of the disease defined as heavy immunodeficiencies known under the abbreviation AIDS (TheNew England Journal of Medicine 313.24, pagg. 1492-1974;, pagg. 1498-1504 pagg. 1504-1510 1985).

-the HSV-1 and HSV-2 viruses to which the nervous and dermal phenomena at the level of genital organs are to be attributed, known under the name of Herpes genitalis (Jama 249 9 pagg. 1041-1049 1982).

The nonylphenoxy polyethoxyethanol (Nonoxynol 9) and components related thereto are surface active substances already widely used as chemical contraceptives for local use.

This surface active substance is also endowed with a relevant antiviral activity, it being capable of inhibiting the infectivity of enveloped viruses, (provided with a peric apcidic envelop,) namely those enveloped from a membrane of cellular origin; among them those responsible of several infections sexually transmitted are included (Herpes Virus 1 and 2, Citomegalovirus, HTLV III).

These viruses, as well as all the other living forms which may become paraxites, are dispersed in the biological fluids which are into contact with the mucosae, such as: mucous, sperma, serum and blood, urines and feces. From the biological liquids the germs may diffuse within the genital organs or, depending on their nature and infectivity, penetrate within the organism, showing their effects also on other organs and apparatuses.

Consequently, if for prophylactic purposes the preparation according to the invention is used, which, during the sexual contacts, by absorbing the biological liquids, can actively eliminate the living forms thereinto, the probabilities of transmission between the two partners are exceedingly reduced and the major risks are eliminated.

The ective drawing of biological liquids, instead of the passive diffusion, obtained with the common local chemical biocides, permits the safety coefficient to be relevantly increased.

As already mentioned, the compositions according to the present invention may be provided in forms having a low prehydration degree, which are thus in solid form with a content of a prehydration water varying between 10% and 100% on dry basis by weight or in form concentrated gels or creams having the above physical characteristics and having a content of prehydration water of 100-1000% by weight on dry basis.

In the case in which said concentrated formulations of creams and gels are prepared with a water content such as they are in form of viscous paste, they can be packaged in a form suitable for the application, such as the so called micro-enemas for the disposable single use microdis pensers, or in form of syringe dispensers or as device having the same function.

In the Italian Patent Application No. 22910 A/80, which is herein referred to for more details, modified dextrane are described, particularly hydrophilic cross linked dextrane.

Such a vehicle can be either partially or totally combined or substituted for with water swellable polymers or vegetal, animal or synthetic gums, such as pectins, guar gum, carrageenates, alginates, carboxymethylstarches, polysaccharide derivatives, starches and their derivatives, carboxyvinyl polymers and the like.

The activity of the nonylphenoxypolyethoxyethanol in turn may be enhanced by adding other spermicides or virulicided such as 8-hydroquinoline, lactic acid, phenylmercuric nitrate or borate, benzetonium chloride, cethylpiridimum chloride, 9-amino acridine, chloramine T, idusidine and their mixtures.

A generic disinfectant activity can be obtained by adding to the formulation, in adequate doses and also in association, the normal disinfectants, bactericides, fungicides, anti-yeasts and anti-protozoa, for instance trichomonacides belonging to several chemical classes.

There are now given some examples of formulations according to the invention, it being understood that the same have only exempliying and non limiting purposes.

1) <u>Spermicide-virulicide</u> <u>tablets</u> <u>for</u> <u>local</u> <u>use</u>

Nonoxynol 9 mg 50
modified dextrane mg 450
microgranular cellulose mg 350
polyglycol 6000 mg 50
water mg 100

2) Spermicide-virulicide microtablets for local use

Nonoxynol 9 mg 2.5
modified dextrane mg 22.5
microgranular cellulose mg 17.7
polyglycol 6000 mg 2.5
water mg 5.0

3) Spermicide-virulicide granulate for local use

Nonoxynol 9 g 10
modified dextrane g 45
microgranular cellulose g 10
polyglycol 6000 g 5
water g 30

4) Spermicide-virulicide concentrated gel for local use

Nonoxynol 9 g 5
modified dextrane G 15
microcrystalline cellulose g 10
water g 30

5) Spermicide-virulicide concentrated cream for local use

Nonoxynol 9 g 10
modified dextrane g 15
self-emulsifying base g 10
water g 65

**Claims**

1. Composition for topical use comprising an active ingredient having spermicide, virulicide, disinfectant, and/or bactericide activity or their combinations, and a hydratable or swellable vehicle, characterized in that said composition is partially prehydrated with water.

2. Composition according to claim 1, wherein said vehicle is selected among modified dextranes and water swellable polymers.

3. Composition according to claim 2, wherein said water swellable polymers are selected in the group comprising pectines, guar gum, carrageenates, alginates, carboxymethylstarches, polysaccharide derivatives, starches and their derivatives, carboxyvinyl polymers and the like.

4. Composition according to claim 1, wherein , in order to obtain fluid formulations, said partial prehydration is carried out with 100 to 1000% of water referred to the dry content of the composition.

5. Composition according to claim 1, wherein in order to obtain solid formulations, said partial prehydration is carried out with 10 to 100% of water referred to the dry content of the composition.

6. Composition according to claim 4, in form of disposable single use enema or of syringe dispenser.

7. Composition according to claim 5, in form of tablets or granulate.

8. Composition according to each of the preceeding claims, wherein said active ingredient is nonylphenoxypolyethoxyethanol.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 472 376 (TOKO YAKUHIN KOGYO KABUSHIKI) <br> * Column 1, lines 47-56; column 3, lines 50-51; column 4, lines 2-27; claims * | 1-7 | A 61 K 47/00 |
| | --- | | |
| Y | WO-A-7 900 014 (VORHAUER LABORATORIES INC.) <br> * Page 6, lines 14-18; page 12, lines 1-26 * | 1-5,8 | |
| | --- | | |
| Y | EP-A-0 065 385 (SYNTEX) <br><br> * Example 1; claims; page 5, line 1; page 3, lines 21-23 * | 1-4,6, 8 | |
| | --- | | |
| Y | WO-A-8 401 502 (A. KOVACS et al.) <br> * Abstract; page 4, lines 13-14 * | 1-3,5, 7 | **TECHNICAL FIELDS SEARCHED (Int. Cl.4)** |
| | --- | | A 61 K 9/00 <br> A 01 N 25/00 |
| A,D | GB-A-2 078 110 (CRINOS INDUSTRIA FARMACOBIOLOGICA) & IT-A-80 22 910 | 1-3 | |
| | ---     -/- | | |

The present search report has been drawn up for all claims

| Place of search | Date of completion of the search | Examiner |
|---|---|---|
| THE HAGUE | 16-10-1987 | GERLI P.F.M. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 90, 1979, page 361, abstract no. 192473j, Columbus, Ohio, US; E. TOUITOU et al.: "New hydrophilic vehicle enabling rectal and vaginal absorption of insulin, heparin, phenol red and gentamicin", & J. PHARM. PHARMACOL. 1978, 30(10), 662-3 * Abstract * | 1-8 | |
| E | EP-A-0 232 830 (CRINOS INDUSTRIA FARMACOBIOLOGICA) * Claims 1-5,7; page 3, lines 8-11; page 5, line 25 - page 6, line 2; page 6, lines 16-18 * | 1-5,7 | |

TECHNICAL FIELDS SEARCHED (Int. Cl.4)

The present search report has been drawn up for all claims

| Place of search | Date of completion of the search | Examiner |
|---|---|---|
| THE HAGUE | 16-10-1987 | GERLI P.F.M. |